(19) Europäisches Patentamt
European Patent Office
Office européen des brevets

(11) Veröffentlichungsnummer : **0 164 045**
**B1**

(12) **EUROPÄISCHE PATENTSCHRIFT**

(45) Veröffentlichungstag der Patentschrift : **05.04.89**

(51) Int. Cl.⁴ : **C 07 C 25/02**, C 07 C 17/00

(21) Anmeldenummer : **85106495.6**

(22) Anmeldetag : **25.05.85**

(54) **Verfahren zur Isomerisierung von Mono- oder Dichlortoluolen.**

(30) Priorität : **02.06.84 DE 3420706**
**13.09.84 DE 3433812**

(43) Veröffentlichungstag der Anmeldung :
**11.12.85 Patentblatt 85/50**

(45) Bekanntmachung des Hinweises auf die Patenterteilung : **05.04.89 Patentblatt 89/14**

(84) Benannte Vertragsstaaten :
**BE CH DE FR GB IT LI NL**

(56) Entgegenhaltungen :
**EP-A- 0 072 008**
**DE-A- 3 334 674**

(73) Patentinhaber : **HOECHST AKTIENGESELLSCHAFT**
**Postfach 80 03 20**
**D-6230 Frankfurt am Main 80 (DE)**

(72) Erfinder : **Eichler, Klaus, Dr.**
**Im Traminer 10**
**D-6236 Eschborn 2 (DE)**
Erfinder : **Arpe, Hans-Jürgen, Prof. Dr.**
**de-Ridder-Weg 10**
**D-6230 Frankfurt am Main 80 (DE)**
Erfinder : **Baltes, Herbert, Dr.**
**Johannesallee 24**
**D-6230 Frankfurt am Main 80 (DE)**
Erfinder : **Leupold, Ernst Ingo, Dr.**
**Am Zäunefeld 15**
**D-6392 Neu-Anspach (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

## Beschreibung

Die vorliegende Erfindung betrifft ein Verfahren zur Isomerisierung von Mono- oder Dichlortoluolen.

Bei der Chlorierung von Toluol in Gegenwart von Katalysatoren entstehen die kernchlorierten isomeren 2-, 3- und 4-Chlortoluole in einem je nach eingesetztem Katalysatortyp und gewählten Reaktionsbedingungen wechselnden Verhältnis. So führt die Chlorierung von Toluol beispielsweise bei 50 °C in Gegenwart von $FeCl_3$ als Katalysator zu einem Produktgemisch aus 19 Gew.-% Toluol, 49 Gew.-% 2-Chlortoluol, 2 Gew.-% 3-Chlortoluol und 25,5 Gew.-% 4-Chlortoluol. Daneben bilden sich zusätzlich 4,5 % Dichlortoluol (Ullmanns Encycl. der techn. Chemie (1975) Band 9, Seite 512). Die Verwendung von $TiCl_4$, $SnCl_4$, $WCl_6$ oder $ZrCl_4$ als Katalysatoren bei einer Chlorierungstemperatur von 10-30 °C erhöht den 2-Chlortoluolanteil auf 75 Gew.-% (US-PS 3 000 975), während der Einsatz von Dischwefeldichlorid und den genannten Metallchloriden oder von Schwefel und $SbCl_3$ das Isomerenverhältnis zugunsten des 4-Chlortoluols verschiebt (US-PS 1 946 040). In keinem Fall gelingt es, durch geeignete Wahl des Katalysators oder der Reaktionsbedingungen zu einem einheitlichen Isomeren zu gelangen. Insbesondere ist reines 3-Chlortoluol nicht durch direkte Toluol-Chlorierung darstellbar.

Es ist bereits bekannt, daß sich Chlortoluole in Gegenwart von $AlCl_3$ (G. A. Olah, M. W. Meyer, J. Org. Chem. 27, 3464 (1964)) oder von $HF/BF_3$ (US-PS 3 742 073) isomerisieren lassen. Dafür werden jedoch erhebliche Katalysatormengen benötigt, und es treten Korrosionsprobleme auf. Auch die Isomerisierung von Chlortoluolen an Zeolith-Katalysatoren wurde bereits beschrieben (EP-A1-46 665 und EP-A1-72 008). Nachteilig wirkt sich dabei die rasche Desaktivierung der Zeolith-Katalysatoren durch Verkokung aus, was zu einem rapiden Abfall des Umsatzes nach kurzer Zeit führt. In EP-A1-62 261 wird zwar ein Verfahren beschrieben, bei dem diese Desaktivierung wesentlich geringer in Erscheinung tritt, es ist aber dafür notwendig, ein organisches Verdünnungsmittel zuzusetzen, was eine technische Durchführung der Isomerisierung sehr aufwendig gestaltet, da dieses Verdünnungsmittel abgetrennt und in den Prozeß zurückgeführt werden muß.

Dichlortoluole stellt man technisch im allgemeinen ebenfalls durch Kernchlorierung von Toluol dar. Dabei entsteht zunächst ein Gemisch aus 2- und 4-Chlortoluol, dessen Weiterchlorierung zu 2,4-Dichlortoluol und — in geringerem Maße — zu 2,3-Dichlortoluol führt (R. Stroh in : Houben-Weyl, Methoden der Organischen Chemie, Band V/3, Halogenverbindungen, Stuttgart 1962, S. 659, 660).

Die anderen Isomeren des Dichlortoluols entstehen bei der Toluol-Chlorierung nur in geringem Maße oder gar nicht (Ullmanns Enzyklopädie der Technischen Chemie, 4. Auflage, Band 9, S. 513). Ihre Herstellung erfordert zum Teil aufwendige und kostspielige mehrstufige Synthesen. So ist das 2,5-Dichlortoluol beispielsweise erhältlich durch Diazotierung nach Sandmeyer aus 5-Chlor-2-aminotoluol oder aus 2,5-Diaminotoluol.

Es ist bereits bekannt, daß sich Dichlortoluole an Zeolith-Katalysatoren isomerisieren lassen (Chemical Abstracts 100, 51224 s). Nachteilig wirkt sich dabei die rasche Desaktivierung der Zeolith-Katalysatoren durch Verkokung aus, was zu einem rapiden Abfall des Umsatzes nach kurzer Zeit führt.

Somit bestand die Aufgabe, ein Verfahren zur Isomerisierung von Mono- oder Dichlortoluolen zu entwickeln, bei dem der Katalysator ohne Zusatz eines organischen Verdünnungsmittels lange Standzeiten, geringe Desaktivierung und hohe Aktivität aufweist.

Es wurde nun gefunden, daß bei der Isomerisierung von Mono- und Dichlortoluolen an zirkonhaltigen Zeolithen vom Pentasil-Typ, wie sie z. B. in EP-A2-77 523 beschrieben werden, höhere Ausbeuten und längere Standzeiten erhalten werden können als mit den bisher beschriebenen Katalysatoren, ohne daß der Zusatz eines organischen Verdünnungsmittels notwendig ist.

Gegenstand der Erfindung ist daher ein Verfahren zur Isomerisierung eines oder mehrerer Monochlortoluole oder eines oder mehrerer Dichlortoluole an einem Zeolith-Katalysator, dadurch gekennzeichnet, daß ein zirkonhaltiger Zeolith vom Pentasil-Typ verwendet wird. Insbesondere ist Gegenstand der vorliegenden Erfindung ein Verfahren zur Herstellung eines Gemisches aus 3- und 4-Chlortoluol durch Isomerisierung von 2-Chlortoluol an einem solchen Zeolithen. Ein weiterer Gegenstand der vorliegenden Erfindung ist ein Verfahren zur Isomerisierung von 2,4-Dichlortoluol oder 3,4-Dichlortoluol an einem derartigen Zeolith-Katalysator.

Aufgrund des Standes der Technik war es überraschend und in keiner Weise vorhersehbar, daß sich an zirkonhaltigen Zeolithen des Pentasil-Typs höhere Isomerisierungsausbeuten bei geringerer Desaktivierung des Katalysators erzielen lassen als mit den vorbeschriebenen zirkonfreien Zeolithen. Der Vergleich zwischen Beispiel 1 und Vergleichsbeispiel 1, sowie zwischen Beispiel 2 und Vergleichsbeispiel 2 zeigt, daß mit den erfindungsgemäßen Katalysatoren unter gleichen Bedingungen erheblich höhere Isomerisierungsausbeuten erhalten werden. Darüberhinaus wird im Beispiel 1 über einen Zeitraum von 90 Stunden keine Änderung der katalytischen Aktivität beobachtet, während mit den bisher bekannten Katalysatoren in einem weit kürzeren Zeitraum bereits ein deutliches Absinken der Aktivität durch Katalysatordesaktivierung stattfindet, wie auch das Vergleichsbeispiel zeigt. Außerdem arbeitet der erfindungsgemäße Katalysator selektiver, da die Nebenproduktbildung geringer ist.

Beispiel 4 zeigt, daß der teilweise desaktivierte Katalysator nach dem Regenerieren wieder die volle Aktivität besitzt. Bei der Isomerisierung des 2,4-Dichlortoluols bilden sich vorwiegend 2,5-Dichlortoluol und 3,4-Dichlortoluol, die Isomerisierung von 3,4-Dichlortoluol führt zu 2,4- und 2,5-Dichlortoluol. Es ist

also möglich, aus 2,4-Dichlortoluol das 2,5-Dichlortoluol herzustellen, wenn man nicht umgesetztes 2,4-Dichlortoluol und das entstandene 3,4-Dichlortoluol in den Reaktor zurückführt.

Zur erfindungsgemäßen Monochlortoluol-Isomerisierung wird 2-, 3- oder 4-Chlortoluol oder ein Gemisch aus zweien oder allen dreien dieser Isomeren mit einem zirkonhaltigen Zeolith-Katalysator vom Pentasil-Typ in Kontakt gebracht. Setzt man ein Gemisch ein, das alle drei Isomeren enthält, so kann natürlich nur dann eine Isomerisierung beobachtet werden, wenn die Zusammensetzung des Ausgangsgemisches bei der Reaktionstemperatur abweicht von der eines Chlortoluol-Gemisches, das sich im thermodynamischen Gleichgewicht befindet.

Zur erfindungsgemäßen Dichlortoluol-Isomerisierung wird ein Dichlortoluol oder ein Gemisch mehrerer isomerer Dichlortoluole entweder alleine oder zusammen mit einem oder mehreren organischen Verdünnungsmitteln gasförmig oder flüssig über den Zeolith-Katalysator geleitet. Als Dichlortoluol wird im allgemeinen das im großtechnischen Maßstab verfügbare 2,4-Dichlortoluol eingesetzt. Aber auch die anderen isomeren Dichlortoluole, beispielsweise das 3,4-Dichlortoluol oder das 2,6-Dichlortoluol, lassen sich nach dem erfindungsgemäßen Verfahren isomerisieren. Eine Isomerisierung kann natürlich wieder nur beobachtet werden, wenn die Zusammensetzung des Ausgangsgemisches von der eines Gemisches, das sich im thermodynamischen Gleichgewicht befindet, abweicht. Als organische Verdünnungsmittel eignen sich insbesondere aromatische Kohlenwasserstoffe, vorzugsweise Chlorbenzol, Benzol und/oder Toluol. Das Molverhältnis des Verdünnungsmittel zu Dichlortoluol beträgt im allgemeinen 0 : 1 bis 10 : 1, vorzugsweise 0 : 1 bis 3 : 1.

Als Katalysatoren für die Isomerisierung der Mono- und Dichlortoluole sind Zirkonsilikate und Zirkonaluminosilikate mit Pentasil-Struktur geeignet.

Für den Begriff Pentasile gilt dabei die Definition von Kokotailo und Meier (« Pentasil family of high silicon crystalline materials » in Special Publication N° 33 of the Chemical Society, London, 1980). Die Pentasil-Familie umfaßt beispielsweise die synthetischen Zeolithe ZSM-5 (US-PS 3 702 886), ZSM-8 (GB-PS 1 334 243), ZSM-11 (US-PS 3 709 979) und ZSM-23 (US-PS 4 076 842).

Beim erfindungsgemäßen Verfahren sind Zirkonosilicate und Zirkonoaluminosilicate mit ZSM-5-Struktur geeignet, vorzugsweise solche mit folgender Zusammensetzung, ausgedrückt in Molverhältnissen der Oxide :

$SiO_2$ : (0-0,15) $Al_2O_3$ : (0,002-1,0) $ZrO_2$, insbesondere
$SiO_2$ : (0-0,1) $Al_2O_3$ : (0,01-0,4) $ZrO_2$ (siehe EP-A2-77523).

Diese zirkonhaltigen Zeolithe lassen sich nach den gleichen Methoden und unter Einsatz der gleichen organischen Verbindungen herstellen, wie sie auch für die Synthese des zirkonfreien Zeolithen ZSM-5 beschrieben wurden, beispielsweise unter Verwendung von

Alkylammoniumverbindungen (US-PS 3 702 886)
Alkylaminen (US-PS 4 151 189)
Alkyldiaminen (DE-OS 2 817 576, DE-OS 2 831 334)
Alkylaminen in Gegenwart von Alkylierungsmitteln (EP-OS 11362, DE-AS 2 212 810)
Aminoalkoholen (GB-PS 2 023 562)
Alkoholen (DE-OS 2 935 123, US-PS 4 175 114, EP-OS 42 225, DE-OS 2 643 929)
Ethern (EP-OS 51 741)

Vorzugsweise verwendet man Alkylammoniumverbindungen, Alkyldiamine, oder Alkylamine in Gegenwart von Alkylierungsmitteln. Unter den Alkylammoniumverbindungen sind besonders bevorzugt die Tetrapropylammoniumverbindungen, beispielsweise das Hydroxid oder eines der Halogenide. Ein besonders geeignetes Alkyldiamin ist Hexamethylendiamin.

Zur Synthese der zirkonhaltigen Pentasile mischt man eine oder mehrere Verbindungen aus den genannten Klassen mit Zirkon-Verbindungen und mit Silizium- und Natriumverbindungen und Wasser — sowie im Falle der Aluminosilicate noch zusätzlich mit Aluminiumverbindungen — und erhitzt dieses Gemisch in einem geschlossenen Gefäß. Dem Gemisch werden vorzugsweise darüberhinaus vor dem Erhitzen Impfkristalle eines Pentasils zugesetzt.

Falls man Tetrapropylammoniumverbindungen verwendet, werden die Ausgangsverbindungen im allgemeinen in folgendem Verhältnis eingesetzt, ausgedrückt in Molverhältnissen der Oxide :

$SiO_2$ : (0-0,2) $Al_2O_3$ : (0,01-1,0) $ZrO_2$ : (0,01-0,5) $Na_2O$ : (0,02-1,0) $R_2O$ : (5-100) $H_2O$,

vorzugsweise im Verhältnis

$SiO_2$ : (0-0,1) $Al_2O_3$ : (0,01-0,4) $ZrO_2$ : (0,02-0,3) $Na_2O$ : (0,03-0,6) $R_2O$ : (10-40) $H_2O$,

wobei R gleich Tetrapropylammonium ist.

Als Silizium-, Aluminium-, Zirkon-, bzw. Natriumverbindungen können beispielsweise eingesetzt werden : Kieselsäuregel, Natriumsilicat, Aluminiumhydroxid, Aluminiumsulfat, Natriumaluminat, Aluminiumhalogenide, Aluminiummetahydroxid, Zirkonhalogenide, Zirkonsulfat, Zirkonylchlorid, Natriumhydroxid, Natriumsulfat, Natriumhalogenide. Aber auch andere Verbindungen der fünf genannten Elemente eignen sich für die Herstellung der Zeolithe.

Das Gemisch der jeweils gewählten Verbindungen mit Wasser wird im allgemeinen 18 bis 360 Stunden, vorzugsweise 24 bis 240 Stunden lang auf eine Temperatur zwischen 100 und 200 °C, vorzugsweise zwischen 130 und 170 °C, in einem geschlossenen Gefäß erhitzt.

Die gebildeten Zeolithe werden in üblicher Weise, z. B. durch Filtration, isoliert, gewaschen und getrocknet.

Bei dem erfindungsgemäßen Verfahren werden die Zeolithe vorzugsweise in ihrer sauren Form eingesetzt. Diese sauren Formen lassen sich nach bekannten Methoden aus den Alkalimetall-Formen, wie sie in der Regel bei der Zeolith-Synthese anfallen bzw. als Naturprodukte vorkommen, durch vollständigen oder partiellen Ionenaustausch herstellen. Eine übliche Methode zur Herstellung der H-Form eines Zeoliths besteht beispielsweise darin, die Alkalimetall-Form zunächst durch partiellen oder vollständigen Ionenaustausch mit einer Ammoniumsalz-Lösung in die Ammoniumform und diese anschließend durch Kalzinieren in die H-Form zu überführen. Aber auch die mit Alkali-, Erdalkali- und mit Seltenerdmetall-Ionen ausgetauschten Formen zeigen katalytische Aktivität.

Die erfindungsgemäßen Zeolith-Katalysatoren bestehen im allgemeinen aus der katalytisch aktiven Zeolith-Komponente sowie einem Bindermaterial. Letzteres ist erforderlich, um den Zeolith in eine für das erfindungsgemäße Verfahren geeignete äußere Form zu bringen.

Als Bindermaterialien eignen sich vor allem Oxide oder Hydroxide des Aluminiums und die Oxide bzw. Hydroxide des Siliciums sowie Schichtsilicate, beispielsweise aus der Kaolin- oder Montmorillonit-Familie.

Dieser so hergestellte Zeolith-Kalalysator wird gewöhnlich vor dem Einsatz in der erfindungsgemäßen Isomerisierungsreaktion zunächst durch Kalzinieren bei Temperaturen zwischen 300 und 700 °C aktiviert. Zur besseren Stabilisierung des Katalysators ist es manchmal vorteilhaft, die Kalzinierung in Gegenwart von Wasserdampf, Ammoniak oder deren Mischungen durchzuführen.

Falls in der Gasphase gearbeitet wird, besteht eine günstige einfache Verfahrensweise zur Durchführung der erfindungsgemäßen Isomerisierung darin, daß man das oder die Mono- oder Dichlortoluole — bei letzteren ggf. auch das Verdünnungsmittel — aus einer Dosiervorrichtung zuerst in eine Verdampfungszone und das entstandene Gas dann durch ein von außen beheiztes und mit dem Katalysator gefülltes Reaktionsrohr leitet. Bei Durchführung der Isomerisierung in flüssiger Phase wird das Einsatzmaterial erst erwärmt und dann in flüssiger Form durch das mit dem Katalysator gefüllte Reaktionsrohr geleitet.

Es hat sich im Hinblick auf die Standzeit des Katalysators als vorteilhaft erwiesen, dem Einsatzmaterial zusätzlich noch Wasserstoff oder Wasserdampf zuzumischen.

Darüberhinaus kann es noch vorteilhaft sein, ein unter den Reaktionsbedingungen inertes Trägergas zuzumischen. Als Trägergase eignen sich z. B. Stickstoff und Edelgase.

Wasserstoff, Wasserdampf und/oder das Trägergas werden dabei in einer solchen Menge zugesetzt, daß die Verweilzeit zwischen einer und hundert Sekunden liegt.

Die Vermischung des Wasserstoffes, Wasserdampfes und/oder des Trägergases mit dem oder den Monochlortoluolen bzw. dem oder den Dichlortoluolen erfolgt am vorteilhaftesten in der Verdampfungs- bzw. Erwärmungszone. Es hat sich dabei als vorteilhaft erwiesen, diese Gase vor der Vermischung auf Reaktionstemperatur aufzuheizen.

Die erfindungsgemäße Isomerisierung wird im allgemeinen bei Temperaturen zwischen 200 und 550 °C, vorzugsweise bei 250 bis 500 °C, sowie Drücken von 0,1 bis 30 bar, vorzugsweise bei 1 bis 20 bar, insbesondere bei Normaldruck durchgeführt. Die Belastung des Zeolith-Katalysators, ausgedrückt als LHSV (Liquid Hourly Space Velocity, $h^{-1}$), liegt im allgemeinen zwischen 0,05 und 10 $h^{-1}$, vorzugsweise zwischen 0,2 und 5 $h^{-1}$.

Die Reaktionsprodukte werden nach Verlassen des Reaktors zur Abtrennung der kondensierbaren Anteile gekühlt. Die erfindungsgemäße Isomerisierung ist jedoch nicht auf diese Verfahrensweise (Festbett-Reaktor) beschränkt, sondern läßt sich im Prinzip auch in anderen geeigneten Reaktor-Typen durchführen (z. B. Wirbelschicht-Reaktor).

Das nicht umgesetzte Ausgangsprodukt kann nach der Trennung durch Destillation, Kristallisation oder selektive Adsorption wieder in den Reaktor zurückgeführt werden.

Sollte die Aktivität des Katalysators mit der Zeit aufgrund einer Verkokung abnehmen, kann er regeneriert werden. Dies geschieht, indem Sauerstoff, Luft, Stickstoff/Luft, Sauerstoff/Luft, Sauerstoff/Inertgas oder Luft/Inertgas bei Temperaturen zwischen 300 und 650 °C über den desaktivierten Katalysator geleitet wird. Stickstoff/Luft wird dabei bevorzugt. Die Temperatur sollte dabei an keiner Stelle des Reaktors 650 °C übersteigen. Nach dem Regenerieren besitzt der Katalysator wieder die volle Aktivität.

Mono- und Dichlortoluole sind wichtige Zwischenprodukte für die Herstellung von Farbstoffen, Pharmazeutika, Konservierungsmitteln und Pflanzenschutzmitteln und dienen außerdem als Ausgangsprodukte für die Herstellung einer Anzahl chlorhaltiger Aromaten.

Die Erfindung soll durch die folgenden Beispiele erläutert werden, wobei die Beispiele aber in keiner Weise einschränkend sein sollen.

## Beispiele

### Beispiel 1
(Isomerisierung von 2-Chlortoluol an zirkonhaltigem Zeolith vom Pentasil-Typ)

a) Katalysator-Herstellung

Zirkonoaluminosilicat vom Pentasil-Typ wurde gemäß Beispiel 1 der EP-A2-77523 wie folgt hergestellt : 16,6 g Natriumaluminat (54 Gew.-% $Al_2O_3$, 41 Gew.-% $Na_2O$) und 14,8 g Natriumhydroxid wurden in 200 g 20 Gew.-%iger wäßriger Tetrapropylammoniumhydroxid-Lösung gelöst (Lösung A). Eine weitere Lösung (Lösung B) wurde hergestellt, indem man 620 g 40 Gew.-%iges kolloidales Kieselgel in 2 300 g 20 Gew.-%iger wäßriger Tetrapropylammoniumhydroxid-Lösung löste und diese Lösung am Rotationsverdampfer auf insgesamt 2 200 g einengte. Lösung A und Lösung B wurden miteinander vermischt. Zu dieser Mischung wurden unter intensivem Rühren 37,8 g Zirkonylchlorid $ZrOCl_2 \cdot 8H_2O$ gegeben. Die entstandene Suspension wurde homogenisiert und in einem geschlossenen Gefäß 120 h auf 160 °C erhitzt. Das entstandene Produkt wurde abfiltriert, mit Wasser gewaschen und bei 120 °C getrocknet. Man erhielt 273 g Zirkonoaluminosilicat. Die Röntgenbeugungsanalyse zeigte ein gut kristallines Produkt mit ZSM-5-Struktur.

Anschließend wurde das Pulver 2 Stunden bei 400 °C, 3 h bei 450 °C und 8 h bei 500 °C an Luft calciniert.

In Molverhältnissen der Oxide ausgedrückt, hatte dieses Material die Zusammensetzung :

$SiO_2 : 0{,}035\ ZrO_2 : 0{,}026\ Al_2O_3 : 0{,}023\ Na_2O$.

Es wurde dreimal für einige Stunden bei 100 °C mit 1-molarer Ammoniumnitratlösung behandelt, gewaschen, getrocknet und einige Stunden bei 500 °C in Luft calciniert.

65 g des so erhaltenen Pulvers wurden mit 35 g $Al_2O_3$ zu Strangpresslingen von 1,6 mm Durchmesser verarbeitet, vier Stunden bei 500 °C calciniert, auf eine Teilchengröße von 0,25 bis 1,0 mm zerkleinert und bei 450 °C im Wasserstoffstrom zwei Stunden lang calciniert.

b) Isomerisierung

15 ml des gemäß a) hergestellten Katalysators wurden in einen Rohrreaktor aus Glas von 16 mm Innendurchmesser und 50 cm Länge eingeführt und mit Glaskugeln zum Verdampfen des flüssigen Ausgangsproduktes überschichtet. Die Temperatur in der Katalysatorschüttung konnte mit einem Thermoelement, das sich in der Mitte des Reaktors befand und in axialer Richtung verschiebbar war, gemessen werden. Der Reaktor und die Verdampfungszone befanden sich in einem elektrisch beheizten Ofen. 6 ml/h 2-Chlortoluol wurden über eine Dosierpumpe dem Reaktor zugeführt. 10 l/h Wasserstoff wurden über eine Gasversorgung, bestehend aus Reduzierventilen und Vorrichtungen zum Messen des Drucks und der Durchflußmenge, ebenfalls über den Katalysator geleitet. Die kondensierbaren Reaktionsprodukte wurden in einer Kühlfalle bei 0 °C kondensiert, ausgewogen und gaschromatographisch analysiert.

(Siehe Tabelle 1 Seite 6 f.)

Tabelle 1 zeigt die Ergebnisse :

| Dauer des Versuchs (h) | Temperatur (°C) | Umsatz 2-Chlortoluol (%) | Selektivitäten 3-Chlortoluol (%) | 4-Chlortoluol (%) | Toluol (%) |
|---|---|---|---|---|---|
| 1 - 20 | 250 - 340 | 15,3* | 82,2* | 12,1* | 5,1* |
| 21 | 370 | 20,3 | 76,1 | 17,0 | 5,3 |
| 46 | 370 | 11,6 | 75,3 | 17,3 | 5,9 |
| 47 | 400 | 35,4 | 68,3 | 21,3 | 5,8 |
| 66 | 400 | 23,5 | 69,4 | 22,9 | 5,8 |
| 89 | 400 | 17,3 | 70,2 | 22,4 | 5,5 |
| 90 | 430 | 42,0 | 64,4 | 21,8 | 8,0 |
| 122 | 430 | 26,9 | 66,3 | 24,3 | 7,0 |
| 148 | 430 | 27,4 | 66,5 | 24,7 | 7,0 |
| 164 | 430 | 27,8 | 67,2 | 25,7 | 5,9 |
| 179 | 430 | 27,0 | 67,0 | 26,2 | 5,6 |
| 194 | 430 | 26,3 | 67,8 | 25,8 | 5,2 |
| 210 | 430 | 24,5 | 66,9 | 26,3 | 5,0 |

Tabelle 1
Isomerisierung von 2-Chlortoluol an zirkonhaltigem Pentasil

*) Mittelwert über 20 Stunden

Wie aus der Tabelle ersichtlich, zeigte der Katalysator zwar anfangs nach jeder Temperaturerhöhung eine Desaktivierung, aber ab der 122.-ten Stunde bis zum Ende des Experiments nach 210 Stunden wurden konstante Aktivität und Selektivität beobachtet. Die Selektivität der Bildung von Toluol liegt im Mittel bei etwa 6 %.

Unter der Selektivität der Bildung von X wird der Anteil des umgesetzten Einsatzproduktes, der zu X regiert, verstanden.

Vergleichsbeispiel 1
(Isomerisierung von 2-Chlortoluol an H-ZSM-5)

Diese Isomerisierung wurde unter den gleichen Bedingungen wie in Beispiel 1 durchgeführt, aber mit 15 ml H-ZSM-5 als Katalysator, dessen Herstellung z. B. in US-PS 3 702 886 beschrieben ist.

Tabelle 2 zeigt die Ergebnisse :

| Dauer des Versuchs (h) | Temperatur (°C) | Umsatz 2-Chlortoluol (%) | Selektivitäten 3-Chlortoluol (%) | 4-Chlortoluol (%) | Toluol (%) |
|---|---|---|---|---|---|
| 1 - 16 | 250 - 370 | 22,4* | 65,3* | 26,0* | 5,2* |
| 17 | 400 | 31,3 | 60,9 | 27,3 | 8,8 |
| 23 | 400 | 16,6 | 55,7 | 34,5 | 8,3 |
| 24 | 430 | 28,6 | 56,6 | 27,2 | 13,0 |
| 30 | 430 | 19,2 | 56,2 | 29,9 | 12,0 |

Tabelle 2
Isomerisierung von 2-Chlortoluol an H-ZSM-5

*) Mittelwert über 16 Stunden

Ein Vergleich mit Beispiel 1 ergibt, daß H-ZSM-5 zwar zu Beginn des Experiments eine vergleichbare Aktivität wie das zirkonhaltige Pentasil besitzt, bei 400 °C aber bereits merklich schneller im Umsatz nachläßt und bei 430 °C sowohl niedrigeren Anfangsumsatz als auch ein schnelles Absinken des Umsatzes weit unter das im Beispiel 1 erreichte konstante Niveau aufweist. Außerdem ist die Selektivität der Bildung des Nebenproduktes Toluol am erfindungsgemäßen Pentasil geringer.

Beispiel 2
(Isomerisierung von 2,4-Dichlortoluol an zirkonhaltigem Pentasil)

a) Katalysator-Herstellung
Zirkonoaluminosilicat vom Pentasil-Typ wurde hergestellt wie in Teil a) von Beispiel 1 beschrieben.
b) Isomerisierung
Die Arbeitsweise war wie in Teil b) von Beispiel 1, nur daß jetzt dem Reaktor 6 ml/h, 2,4-Dichlortoluol anstelle von 2-Chlortoluol zugeführt wurden.

Tabelle 3 zeigt die Ergebnisse :

Produktgehalt in Gew.-%

| Dauer (h) | Temperatur (°C) | 2,4-DCT | 2,5-DCT | 3,4-DCT | 2,3-DCT | 3,5-DCT | CT | o-DCB | m-DCB | p-DCB |
|---|---|---|---|---|---|---|---|---|---|---|
| 4 | 400 | 59,4 | 25,8 | 7,0 | < 0,1 | < 0,1 | 1,4 | < 0,1 | 6,9 | <0,1 |
| 6 | 400 | 62,8 | 23,9 | 5,2 | < 0,1 | < 0,1 | 1,3 | < 0,1 | 5,2 | <0,1 |
| 8 | 400 | 64,5 | 23,1 | 6,0 | < 0,1 | < 0,1 | 1,1 | < 0,1 | 4,9 | <0,1 |
| 10 | 400 | 67,7 | 20,9 | 5,6 | < 0,1 | < 0,1 | 0,9 | < 0,1 | 4,6 | <0,1 |
| 12 | 400 | 69,4 | 19,9 | 5,2 | < 0,1 | < 0,1 | 0,9 | < 0,1 | 4,3 | <0,1 |
| 14 | 400 | 68,9 | 20,5 | 5,3 | < 0,1 | < 0,1 | 0,8 | < 0,1 | 4,2 | <0,1 |
| 20 | 400 | 73,4 | 17,6 | 4,6 | < 0,1 | < 0,1 | 0,6 | < 0,1 | 3,5 | <0,1 |

Tabelle 3
Isomerisierung von 2,4-Dichlortoluol an zirkonhaltigem Pentasil
DCT : Dichlortoluol
CT : Chlortoluol
DCB : Dichlorbenzol

Vergleichsbeispiel 2
(Isomerisierung von 2,4-Dichlortoluol an H-ZSM-5)

Diese Isomerisierung wurde unter den gleichen Bedingungen wie in Beispiel 2 durchgeführt, aber mit 15 ml H-ZSM-5 als Katalysator, dessen Herstellung in US-PS 3 702 886, Beispiel 1, beschrieben ist.
Tabelle 4 zeigt die Ergebnisse.
Ein Vergleich mit Beispiel 2 ergibt, daß H-ZSM-5 zwar zu Beginn des Experiments eine vergleichbare Aktivität wie das zirkonhaltige Pentasil besitzt, der 2,4-Dichlortoluol-Umsatz und die Ausbeute an 2,5-Dichlortoluol und 3,4-Dichlortoluol nehmen jedoch viel rascher ab als beim Einsatz des erfindungsgemäßen zirkonhaltigen Zeolithen vom Pentasil-Typ.

(Siehe Tabelle 4 Seite 8 f.)

Produktgehalt in Gew.-%

| Dauer (h) | Temperatur (°C) | 2,4-DCT | 2,5-DCT | 3,4-DCT | 2,3-DCT | 3,5-DCT | CT | o-DCB | m-DCB | p-DCB |
|---|---|---|---|---|---|---|---|---|---|---|
| 1 | 400 | 36,9 | 29,9 | 8,9 | < 0,1 | < 0,1 | 7,4 | < 0,1 | 7,0 | 5,3 |
| 2 | 400 | 48,1 | 28,3 | 6,8 | < 0,1 | < 0,1 | 5,1 | < 0,1 | 5,9 | 3,7 |
| 4 | 400 | 61,9 | 22,0 | 5,7 | < 0,1 | < 0,1 | 2,8 | < 0,1 | 4,2 | 2,3 |
| 6 | 400 | 69,7 | 18,0 | 4,9 | < 0,1 | < 0,1 | 1,7 | < 0,1 | 3,2 | 1,6 |
| 8 | 400 | 74,5 | 15,4 | 4,4 | < 0,1 | < 0,1 | 1,2 | < 0,1 | 2,5 | 1,3 |
| 10 | 400 | 77,5 | 13,6 | 4,0 | < 0,1 | < 0,1 | 0,6 | < 0,1 | 2,1 | 1,0 |
| 12 | 400 | 80,6 | 11,9 | 3,6 | < 0,1 | < 0,1 | 0,6 | < 0,1 | 1,9 | 0,9 |
| 14 | 400 | 80,4 | 12,1 | 3,7 | < 0,1 | < 0,1 | 0,5 | < 0,1 | 1,8 | 0,9 |
| 20 | 400 | 91,2 | 5,6 | 1,7 | < 0,1 | < 0,1 | 0,1 | < 0,1 | 0,7 | 0,3 |

Tabelle 4
Isomerisierung von 2,4-Dichlortoluol an H-ZSM-5

DCT : Dichlortoluol
CT : Chlortoluol
DCB : Dichlorbenzol

Beispiel 3
(Isomerisierung von 3,4-Dichlortoluol an regeneriertem zirkonhaltigem Pentasil)

Der in Beispiel 2 bereits verwendete Katalysator wurde 2 Stunden lang bei 600 °C in Luftatmosphäre regeneriert.

Anschließend wurde mit diesem Katalysator der in Beispiel 2 beschriebene Versuch mit 3,4-Dichlortoluol statt 2,4-Dichlortoluol als Einsatzmaterial durchgeführt. Tabelle 5 zeigt, daß 3,4-Dichlortoluol ebenfalls an zirkonhaltigem Zeolith vom Pentasil-Typ isomerisierbar ist.

Produktgehalt in Gew.-%

| Dauer (h) | Temperatur (°C) | 3,4-DCT | 2,5-DCT | 2,4-DCT | 2,3-DCT | 3,5-DCT | CT | o-DCB | m-DCB | p-DCB |
|---|---|---|---|---|---|---|---|---|---|---|
| 1 | 400 | 29,7 | 26,7 | 23,0 | < 0,1 | < 0,1 | 3,5 | < 0,1 | 8,3 | 4,6 |
| 2 | 400 | 37,8 | 22,9 | 18,6 | < 0,1 | < 0,1 | 2,9 | < 0,1 | 9,9 | 5,1 |
| 4 | 400 | 57,8 | 12,6 | 9,9 | < 0,1 | < 0,1 | 2,2 | < 0,1 | 10,7 | 4,2 |
| 6 | 400 | 67,3 | 8,8 | 6,5 | < 0,1 | < 0,1 | 1,6 | < 0,1 | 10,5 | 3,2 |
| 8 | 400 | 72,3 | 6,8 | 5,2 | < 0,1 | < 0,1 | 1,3 | < 0,1 | 10,1 | 2,6 |
| 10 | 400 | 79,2 | 4,7 | 3,5 | < 0,1 | < 0,1 | 0,9 | < 0,1 | 8,8 | 1,8 |
| 12 | 400 | 85,6 | 3,0 | 2,1 | < 0,1 | < 0,1 | 0,6 | < 0,1 | 6,7 | 1,1 |

Tabelle 5
Isomerisierung von 3,4-Dichlortoluol an regeneriertem zirkonhaltigem Pentasil

DCT : Dichlortoluol
CT : Chlortoluol
DCB : Dichlorbenzol

Beispiel 4
(Isomerisierung von 2,4-Dichlortoluol an regeneriertem zirkonhaltigem Pentasil)

Der in Beispiel 2 und 3 bereits verwendete Katalysator wurde 2 Stunden lang bei 600 °C in Luftatmosphäre regeneriert. Anschließend wurde mit diesem Katalysator der in Beispiel 2 beschriebene Versuch wiederholt. Wie Tabelle 6 zeigt, besitzt der erfindungsgemäße Katalysator nach der Regenerierung die gleiche Aktivität und ergibt die gleiche Produktverteilung wie ein frischer Katalysator.

Produktgehalt in Gew.-%

| Dauer (h) | Temperatur (°C) | 2,4-DCT | 2,5-DCT | 3,4-DCT | 2,3-DCT | 3,5-DCT | CT | o-DCB | m-DCB | p-DCB |
|---|---|---|---|---|---|---|---|---|---|---|
| 1 | 400 | 53,0 | 31,1 | 8,2 | < 0,1 | < 0,1 | 1,9 | < 0,1 | <0,1 | 0,2 |
| 2 | 400 | 57,0 | 28,8 | 7,8 | < 0,1 | < 0,1 | 1,5 | < 0,1 | <0,1 | 0,2 |
| 4 | 400 | 62,7 | 25,3 | 6,9 | < 0,1 | < 0,1 | 1,3 | < 0,1 | <0,1 | <0,1 |
| 6 | 400 | 66,3 | 23,1 | 6,4 | < 0,1 | < 0,1 | 1,0 | < 0,1 | <0,1 | <0,1 |
| 8 | 400 | 69,1 | 21,2 | 6,0 | < 0,1 | < 0,1 | 2,8 | < 0,1 | <0,1 | <0,1 |
| 10 | 400 | 68,0 | 22,2 | 5,5 | < 0,1 | < 0,1 | 3,3 | < 0,1 | <0,1 | <0,1 |
| 12 | 400 | 72,6 | 18,9 | 5,4 | < 0,1 | < 0,1 | 2,3 | < 0,1 | <0,1 | <0,1 |
| 14 | 400 | 72,5 | 19,0 | 5,6 | < 0,1 | < 0,1 | 2,3 | < 0,1 | <0,1 | <0,1 |
| 16 | 400 | 72,7 | 18,9 | 5,6 | < 0,1 | < 0,1 | 2,2 | < 0,1 | <0,1 | <0,1 |

Tabelle 6
Isomerisierung von 2,4-Dichlortoluol an regeneriertem zirkonhaltigem Pentasil
DCT : Dichlortoluol
CT : Chlortoluol
DCB : Dichlorbenzol

## Patentansprüche

1. Verfahren zur Isomerisierung eines oder mehrerer Monochlortoluole oder eines oder mehrerer Dichlortoluole an einem Zeolith-Katalysator, dadurch gekennzeichnet, daß ein zirkonhaltiger Zeolith vom Pentasil-Typ verwendet wird.
2. Verfahren zur Isomerisierung eines oder mehrerer Monochlortoluole an einem Zeolith-Katalysator, dadurch gekennzeichnet, daß ein zirkonhaltiger Zeolith vom Pentasil-Typ verwendet wird.
3. Verfahren zur Herstellung eines Gemisches aus 3- und 4-Chlortoluol durch Isomerisierung von 2-Chlortoluol an einem Zeolith-Katalysator, dadurch gekennzeichnet, daß ein zirkonhaltiger Zeolith vom Pentasil-Typ verwendet wird.
4. Verfahren zur Isomerisierung eines oder mehrerer Dichlortoluole an einem Zeolith-Katalysator, dadurch gekennzeichnet, daß ein zirkonhaltiger Zeolith vom Pentasil-Typ verwendet wird.
5. Verfahren zur Isomerisierung von 2,4-Dichlortoluol an einem Zeolith-Katalysator, dadurch gekennzeichnet, daß ein zirkonhaltiger Zeolith vom Pentasil-Typ verwendet wird.
6. Verfahren zur Isomerisierung von 3,4-Dichlortoluol an einem Zeolith-Katalysator, dadurch gekennzeichnet, daß ein zirkonhaltiger Zeolith vom Pentasil-Typ verwendet wird.
7. Verfahren nach einem der Ansprüche 1 bis 6, dadurch gekennzeichnet, daß der Zeolith Protonen als Kationen enthält.
8. Verfahren nach einem der Ansprüche 1 bis 7, dadurch gekennzeichnet, daß man bei Temperaturen zwischen 200 und 550 °C arbeitet.
9. Verfahren nach einem der Ansprüche 1 bis 8, dadurch gekennzeichnet, daß man bei einem Druck zwischen 0,1 bar und 30 bar arbeitet.
10. Verfahren nach einem der Ansprüche 1 bis 9, dadurch gekennzeichnet, daß die Isomerisierung in Gegenwart von Wasserstoff, Stickstoff, Wasserdampf, Argon oder einem Gemisch aus diesen durchgeführt wird.

## Claims

1. A process for isomerizing one or more monochlorotoluenes or one or more dichlorotoluenes on a zeolite catalyst, which comprises using a zirconium-containing zeolite of the Pentasil type.

2. A process for isomerizing one or more monochlorotoluenes on a zeolite catalyst, which comprises using a zirconium-containing zeolite of the Pentasil type.

3. A process for the preparation of a mixture of 3-chlorotoluene and 4-chlorotoluene by isomerizing 2-chlorotoluene on a zeolite catalyst, which comprises using a zirconium-containing zeolite of the Pentasil type.

4. A process for isomerizing one or more dichlorotoluenes on a zeolite catalyst, which comprises using a zirconium-containing zeolite of the Pentasil type.

5. A process for isomerizing 2,4-dichlorotoluene on a zeolite catalyst, which comprises using a zirconium-containing zeolite of the Pentasil type.

6. A process for isomerizing 3,4-dichlorotoluene on a zeolite catalyst, which comprises using a zirconium-containing zeolite of the Pentasil type.

7. The process as claimed in any of the claims 1-6 wherein the zeolite contains protons as cations.

8. The process as claimed in any of the claims 1-7 wherein the reaction is carried out at temperatures between 200 and 550 °C.

9. The process as claimed in any of the claims 1-8 wherein the reaction is carried out under a pressure between 0.1 bar and 30 bar.

10. The process as claimed in any of the claims 1-9 wherein the isomerization is carried out in the presence of hydrogen, nitrogen, steam, argon or a mixture thereof.

## Revendications

1. Procédé pour isomériser un ou plusieurs monochloro-toluènes ou un ou plusieurs dichloro-toluènes en présence d'un catalyseur à base de zéolite, procédé caractérisé en ce qu'on utilise une zéolite contenant du zirconium du type pentasil.

2. Procédé pour isomériser un ou plusieurs monochloro-toluènes en présence d'un catalyseur à base de zéolite, procédé caractérisé en ce qu'on utilise une zéolite contenant du zirconium du type pentasil.

3. Procédé pour préparer un mélange de chloro-3 toluène et de chloro-4 toluène par isomérisation du chloro-2 toluène en présence d'un catalyseur à base de zéolite, procédé caractérisé en ce qu'on utilise une zéolite contenant du zirconium du type pentasil.

4. Procédé pour isomériser un ou plusieurs dichloro-toluènes en présence d'un catalyseur à base de zéolite, procédé caractérisé en ce qu'on utilise une zéolite contenant du zirconium du type pentasil.

5. Procédé pour isomériser le dichloro-2,4 toluène en présence d'un catalyseur à base de zéolite, procédé caractérisé en ce qu'on utilise une zéolite contenant du zirconium du type pentasil.

6. Procédé pour isomériser le dichloro-3,4 toluène en présence de catalyseur à base de zéolite, procédé caractérisé en ce qu'on utilise une zéolite contenant du zirconium du type pentasil.

7. Procédé selon l'une quelconque des revendications 1 à 6, caractérisé en ce que la zéolite contient, comme cations, des protons.

8. Procédé selon l'une quelconque des revendications 1 à 7, caractérisé en ce qu'on opère à des températures comprises entre 200 et 550 °C.

9. Procédé selon l'une quelconque des revendications 1 à 8, caractérisé en ce qu'on opère sous une pression comprise entre 0,1 bar et 30 bar.

10. Procédé selon l'une quelconque des revendications 1 à 9, caractérisé en ce qu'on effectue l'isomérisation en présence d'hydrogène, d'azote, de vapeur d'eau, d'argon ou d'un mélange de ces corps.